# EUROPÄISCHE PATENTSCHRIFT

(11) **EP 1 053 754 B1**
(45) Veröffentlichungstag und Bekanntmachung des Hinweises auf die Patenterteilung: **22.12.2004**
(21) Anmeldenummer: 00109780.7
(22) Anmeldetag: 09.05.2000
(51) Int. Cl.: A61L 2/00, C07K 1/36, C07K 14/745

(54) **Verfahren zur Inaktivierung von Viren**
Method of inactivating viruses
Méthode d'inactivation de virus

(30) Priorität: 19.05.1999 DE 19923027
(43) Veröffentlichungstag der Anmeldung: 22.11.2000
(73) Patentinhaber: ZLB Behring GmbH, 35041 Marburg (DE)
(72) Erfinder: Schüler, Eckhard, 35041 Marburg (DE); Kumpe, Gerhardt, 35083 Wetter (DE); Nowak, Thomas, 35460 Staufenberg (DE)

(56) Entgegenhaltungen:
- EP-A- 0 124 506
- EP-B- 0 683 678
- US-A- 4 749 783
- US-A- 5 610 147

## Beschreibung

Gegenstand der Erfindung ist ein Verfahren zur Inaktivierung und/oder Eliminierung von umhüllten und/oder nicht-umhüllten Viren aus einer Plasmaproteinlösung.

Das menschliche Plasma ist bekanntlich ein Rohstoff für eine Reihe moderner Therapeutika. Hierzu gehören das Albumin, die Immunglobuline und die Gruppe der Blutgerinnungsfaktoren.

Der Bedarf an Blutgerinnungsfaktoren ergibt sich aus der Notwendigkeit, dass Patienten mit kongenitalen Mängeln einzelner Gerinnungsfaktoren nur durch Substitution dieser Faktoren überleben können. Hinzu kommen aber auch erworbene Mängel an Blutgerinnungsfaktoren, die ebenfalls eine Substitution erforderlich machen.

Obwohl man zunächst davon ausgehen konnte, dass die Substitution von Plasmaproteinen für den Patienten ohne Risiko ist, stellte sich bald heraus, dass durch eine Therapie mit derartigen Präparaten Infektionskrankheiten vom Plasmaspender übertragen werden können, die in vielen Fällen durch Verunreinigungen mit krankheitsauslösenden Viren verursacht werden. Es stellte sich deshalb die Aufgabe, durch Inaktivierung und/oder Eliminierung derartiger Viren hochgereinigte Plasmapräparate und Zubereitungen von Blutgerinnungsfaktoren zur Verfügung zu stellen, die ohne Gefahr einer Virusinfektion beim Patienten angewendet werden können. Hierfür sind bereits mehrere Verfahren vorgeschlagen worden.

So ist aus der europäischen Patentschrift 0 124 506 ein Verfahren zur Inaktivierung von vermehrungsfähigen Krankheitserregern in Präparationen, die plasmatische Enzyme, Gerinnungsfaktoren, Immunglobuline oder sonstige im Blut vorhandene Proteine enthalten, bekannt, bei dem die Präparation durch Zusatz von Ammoniumsulfat auf eine Salzkonzentration von mehr als 0,5 molar gebracht und wärmebehandelt wird, worauf das Salz aus der Präparation entfernt wird. Hierbei wird einer Präparation mit einem Proteingehalt von bis zu 30 % Ammoniumsulfat zugesetzt, bis eine 2- bis 4-molare Konzentration erreicht ist. Dann wird eine Wärmebehandlung während einer Dauer von bis zu 100 Stunden und bei einer Temperatur von 40 bis 121°C durchgeführt. Selbstverständlich muß bei einem derartigen Verfahren darauf geachtet werden, dass bei der angewendeten Salz- und Wärmebehandlung nicht nur die Viren inaktiviert und/oder eliminiert werden, sondern dass dabei auch die biologische Aktivität der in der Präparation enthaltenen Plasmaproteine in möglichst hohem Maße erhalten bleibt. Es besteht deshalb weiterhin ein Bedarf an Verfahren zur Inaktivierung und/oder Eliminierung von Viren aus einer Plasmaproteinlösung, die nicht nur die Übertragung von Virusinfektionen durch derartige Plasmapräparate völlig ausschließen, sondern darüber hinaus so schonend sind, dass sie die biologischen Aktivitäten der Plasmaproteine, insbesondere auch der Blutgerinnungsfaktoren, nicht beeinträchtigen.

Es wurde nun gefunden, dass diese Forderungen in ausgezeichneter Weise durch ein Verfahren zur Inaktivierung und/oder Eliminierung von umhüllten und/oder nicht-umhüllten Viren aus einer Plasmaproteinlösung erfüllt werden, wenn man eine Plasmaproteinlösung bei Raumtemperatur und alkalischen pH-Werten bei einer Ammoniumsalzkonzentration von 13 bis 22 Gew.-% (= 25 bis 39% Ammoniumsulfat-Sättigung) einer Inkubation unterwirft, nach Abtrennung der Ausfällungen und der Entfernung des Ammoniumsalzes bis auf einen Restgehalt von weniger als 0,5 mol/l mehrere Stunden bei etwa 60°C pasteurisiert und sie dann zu einer therapeutisch einsetzbaren Plasmaproteinzubereitung verarbeitet.

Plasmaproteine im Sinne der Erfindung sind nicht nur die intakten, aktiven Proteine selbst, sondern auch deren Vorstufen. Die Plasmaproteine können aus Plasma oder anderen Körperflüssigkeiten gewonnen sein.

Für dieses Verfahren wird vorzugsweise eine durch Affini-tätschromatographie vorgereinigte Plasmaproteinlösung eingesetzt und bei pH-Werten zwischen 8 und 11 gearbeitet. Die Virusreduktion kommt dabei entweder durch Eliminierung, z.B. bei dem caninen Parvovirus (CPV), wobei eine Temperatur von 25 +/- 1°C ganz besonders bevorzugt ist, oder durch eine Kombination von Eliminierung und Inaktivierung, z.B. bei dem bovinen Virus-Diarrhöe-Virus (BVDV) oder bei HIV zustande.

Eine weitere Verbesserung des erfindungsgemäßen Verfahrens läßt sich dadurch erreichen, dass nach der Inkubation mit einer Ammoniumsalzkonzentration von 13 bis 22 Gew.-% (= 25 bis 39% Ammoniumsulfat-Sättigung) und Abtrennung und Ausfällung der Verunreinigungen durch Zentrifugation oder Filtration erneut Ammoniumsalz zugegeben und die Konzentration auf 24 bis 27 Gew.-% (= 44,4 bis 50% Ammoniumsulfat-Sättigung) gesteigert wird. Bei dieser zweiten Fällung wird das Plasmaprotein in reiner Form ausgefällt und dann nach Entfernung des Ammoniumsalzes bis auf einen Restgehalt von weniger als 0,5 mol/l mehrere Stunden bei etwa 60°C pasteurisiert. Das Präzipitat kann dann zu einer therapeutisch einsetzbaren Plasmaproteinzubereitung verarbeitet werden.

Sowohl für die erste als auch für die zweite Inkubation sollten 2 bis 4 Stunden aufgewendet werden. Als Ammoniumsalz wird vorzugsweise Ammoniumsulfat eingesetzt.

Um eine möglichst hohe Restaktivität der Plasmaproteine zu erhalten, empfiehlt es sich, die Pasteurisierung in Gegenwart von Stabilisatoren durchzuführen. Als geeignete Stabilisatoren hat sich eine Mischung von Saccharose (1000 g/l) mit Glycin (150 g/l) erwiesen. Auch in Gegenwart von Saccharose und Kaliumacetat als Stabilisatoren läßt sich eine hohe Restaktivität der Plasmaproteine erhalten. Im allgemeinen wird das erfindungsgemäße Verfahren mit aus Blut gewonnenen Plasmaproteinen durchgeführt und ergibt eine Restaktivität von bis zu 95% (bezogen auf Antithrombin III). Es kann jedoch ebenso auch für biotechnologisch hergestellte, rekombinante bzw. transgene Plasmaproteine eingesetzt werden, die aus Einzellern oder vor allem auch aus transgenen Tieren gewonnen werden.

Besondere Bedeutung hat für das erfindungsgemäße Verfahren auch die Einhaltung alkalischer pH-Werte. Erhöht man unter ansonsten gleichen Bedingungen den pH-Wert, so kann man mit steigendem pH-Wert bei der durch die Fällungsreaktion erreichten Proteinreinigung eine Erhöhung der Virusinaktivierung beobachten. Die erhöhte Virusreduktion ist sowohl für umhüllte als auch für nicht-umhüllte Viren festzustellen und wird auf die Kombination der Wirkungen von Ammoniumsalz und alkalischem pH-Wert zurückgeführt.

Für das erfindungsgemäße Verfahren ist es somit kennzeichnend, dass es zwei virusreduzierende Verfahrensschritte enthält, nämlich die Pasteurisierung in wässriger Lösung (10 Stunden, 60°C) und die Ammoniumsulfatfällung. Beide Virusinaktivierungsschritte verfügen über hohe Reduktionsfaktoren bei der Virusreduktion. Von besonderer Wirkung ist die Wirkung beider Schritte auch auf nicht-umhüllte Viren.

Am Beispiel des Antithrombins III (AT III) läßt sich das erfindungsgemäße Verfahren in folgender Weise beschreiben:

Die Isolierung von humanem Antithrombin III erfolgt aus Spenderblut, welches nach Kryopräzipitation durch Zentrifugieren in corpuskuläre und plasmatische Bestandteile aufgetrennt wird. Der bei der Cohn-Fraktionierung gewonnene 8%-Ethanol-Überstand wird mit einer Heparin-Fractogel-Chromatographiesäule vorgereinigt. Dabei wird AT III auf der Säule angereichert. Das auf der Säule fixierte Heparin bindet sowohl AT III als auch einige andere Plasmaproteine. Die Mehrzahl der lose gebundenen Plasmaproteine wird mit einer Pufferlösung mit niedriger NaCl-Konzentration ausgewaschen, während anschließend AT III mit einer Pufferlösung bestehend aus 2 M NaCl, 50 mM NaH₂PO₄ und einem pH-Wert von 7,5 eluiert wird. Die hohe Reinheit von AT III, die bereits durch diese spezifische Affinitätschromatographie erreicht wird, wird weiter durch die Beseitigung kontaminierender Proteine durch fraktionierte Ammoniumsulfatfällung verbessert. Anschließend wird im Eluat durch Zugabe von festem Ammoniumsulfat eine Konzentration von 31,3 % Ammoniumsulfat-Sättigung eingestellt. Während einer dreistündigen Inkubation bei Raumtemperatur (25°C +/-1) entsteht ein Niederschlag, der durch Zentrifugation oder Filtration abgetrennt und verworfen wird. Zu starkes Rühren ist bei der Inkubation zu vermeiden, weil sonst das Präzipitat zu fein wird und den Filter verstopft. Bei der Inkubation wird ein pH-Wert von 9,0 aufrechterhalten. Anschließend wird die Konzentration von Ammoniumsulfat durch Dialyse bis auf einen Restgehalt von weniger als 0,5 mol/l reduziert und Saccharose (1000 g/l) und Glycin (150 g/l) als Stabilisatoren zugegeben. Danach wird die wässrige, stabilisierte AT III-Lösung bei 60°C 10 Stunden pasteurisiert.

Die hohe Reinheit des Endproduktes konnte durch Zonenelektrophorese und Polyacrylamidgel-Elektrophorese belegt werden.

Zur Untersuchung der Virusabreicherung werden vor der Ammoniumsulfat-Behandlung Viren zugegeben (0,05 Volumenanteile). Dabei wurden folgende Werte für die Virusabreicherung gefunden:

**Tabelle I**

| **Virus** | **Virusreduktionsfaktor (log**_{**10**}**)** |
|---|---|
| HIV | ≥ 7,8 |
| HAV (Hepatitis A Virus) | ≥ 6,1 |
| PRV (Pseudorabies Virus) | ≥ 7,7 |
| BVDV (Boviner Virus-Diarrhöe-Virus | ≥ 7,1 |
| CPV (Caniner Parvovirus) | ≥ 8,1 |

An die erste Ammoniumsulfatfällung kann sich vorzugsweise noch eine zweite Ammoniumsulfatfällung anschließen, bei der die Konzentration durch Zugabe von festem Ammoniumsulfat bis zu einer Endkonzentration von 45,9 % Ammoniumsulfat-Sättigung eingestellt wird. Nach einer Fällzeit von drei Stunden bei Raumtemperatur wird das ausgefällte AT III abgetrennt. Das so gewonnene Präzipitat wird anschließend durch Lösen und Dialyse auf einen Puffer von 0,8 % (w/v) NaCl und 0,8 % (w/v) Ammoniumsulfat eingestellt.

Zur Untersuchung der Virusinaktivierung durch die Pasteurisierung wurden dem Produkt unmittelbar vor der Pasteurisierung infektiöse Viren zugesetzt und anschließend die Probe einer Hitzebehandlung bei 60°C ausgesetzt. Es konnte beobachtet werden, dass alle untersuchten Viren (mit Ausnahme von CPV) nach einer Hitzebehandlung von weniger als 6 Stunden vollständig inaktiviert waren.

Die vom Committee for Proprietary Medicinal Products (CPMP) herausgegebenen Leitlinien für die Europäische Union fordern die stufenweise Untersuchung der Viruseliminierung/-inaktivierung durch den Herstellungsprozess eines Humanplasmaproteins. Nach diesen Richtlinien sollen für derartige Untersuchungen mindestens drei Virusarten verwendet werden, nämlich HIV als relevantes Risikovirus, ein weiteres umhülltes Virus und ein nicht-umhülltes Virus. Die vorstehende Tabelle zeigt, dass hervorragende Reduktionsfaktoren für die unterschiedlichsten Virenarten mit dem erfindungsgemäßen Verfahren erzielt werden konnten.

Zur Untersuchung der Virussicherheit wurden die nach dem erfindungsgemäßen Verfahren hergestellten AT III-Zubereitungen einer klinischen Studie unterworfen. In diese Studie wurden 13 gesunde männliche Probanden aufgenommen, die zuvor weder Bluttransfusionen noch Blutderivate erhalten hatten und bei denen keine Vorgeschichte einer Lebererkrankung vorlag. 7 Probanden waren gegen Hepatitis B geimpft und besaßen daher schützende Antikörper des Typs Anti-HBs. Die übrigen 6 Teilnehmer wiesen einen negativen Befund auf Hepatitis-B-Serummarker auf. Eine Hepatitis-B-Sicherheit ließ sich daher nur bei diesen 6 Probanden verfolgen, während alle 13 Probanden im Hinblick auf die Hepatitis-C- und HIV-Infektionen bewertbar waren. Jeder Teilnehmer dieser Studie erhielt auf randomisierter Basis zwei unterschiedliche Chargen des pasteurisierten AT III-Konzentrats und wurde mit zwei verschiedenen Dosierungen behandelt; d.h. 8 erhielten festgelegte Dosen von 1000 Einheiten, während die übrigen 5 jeweils 50 Einheiten pro kg Körpergewicht erhielt. Im Durchschnitt betrug die an letztere Gruppe verabreichte AT III-Menge etwa 3600 Einheiten pro Person. Alle Probanden wurden während der ersten sechs Monate in zweiwöchigen Abständen und danach alle vier Wochen auf Transaminasen untersucht. Die Übertragung eines Hepatitis-Non-A-Non-B-Virus wurde als bestätigt angesehen, wenn der Serumtransaminasenspiegel bei zwei aufeinanderfolgenden Untersuchungen das 2,5fache des oberen normalen Grenzwertes überschritt und wenn alle anderen Hepatitis-Viren ausgeschlossen werden konnten. Im Nachhinein wurden die Serumproben jedes Probanden auch auf Anti-HCV- und Anti-HIV-2-Antikörper untersucht. Hepatitis-B-Serummarker (HBsAg, Anti-HBc, Anti-HBs) und Anti-HIV 1 wurden alle zwei Monate geprüft.

Nach einer Beobachtungszeit von 12 Monaten wurden bei keinem der 13 Probanden erhöhte Serumtransaminaseaktivitäten festgestellt, und keiner wies einen positiven Befund auf Anti-HIV oder Anti-HCV auf. Darüber hinaus entwickelte keiner der 6 Probanden, die nicht mit Hepatitis-B-Impfstoff geimpft waren, positive HIV-Marker.

Aufgrund dieser klinischen Studie, die nach den Empfehlungen des International Committee of Thrombosis and Hemostasis (ICTH) durchgeführt wurde, und aufgrund der retrospektiven Analyse von Patienten, die mit dem nach dem erfindungsgemäßen Verfahren hergestellten AT III-Konzentrat behandelt waren, kann gefolgert werden, dass das nach dem erfindungsgemäßen Verfahren hergestellte AT III-Präparat nicht mit dem Risiko einer HBV-, HCV- oder HIV-Kontamination behaftet ist.

Die mit dem untersuchten AT III-Präparat gewonnenen experimentellen Daten zeigen, dass das resultierende, pasteurisierte AT III-Präparat fast bis zur Homogenität gereinigt ist und dass dieses Produkt eine hohe Sicherheitsmarge erreicht, weil Viren in verschiedenen Phasen des Herstellungsprozesses effektiv eliminiert und inaktiviert werden. Durch Kombination von Fällungsreaktionen und Pasteurisierung wird dabei eine Virusreduktion erreicht, die einen extrem hohen Sicherheitsstandard der so hergestellten Plasmaproteinzubereitungen gewährleistet.

Dabei ist hervorzuheben, dass das erfindungsgemäße Verfahren durch die Entfernung des Ammoniumsalzes aus der durch die Fällungsreaktion gereinigten Proteinlösung auf einen Restgehalt von weniger als 0,5 mol/l besonders schonend ist und gewährleistet, dass bei der anschließenden Pasteurisierung besonders hohe Restaktivitäten der Plasmaproteine, erhalten bleiben.

Das erfindungsgemäße Verfahren führte bei den in der Tabelle II genannten Plasmaproteinen zu den dort genannten hohen Restaktivitäten:

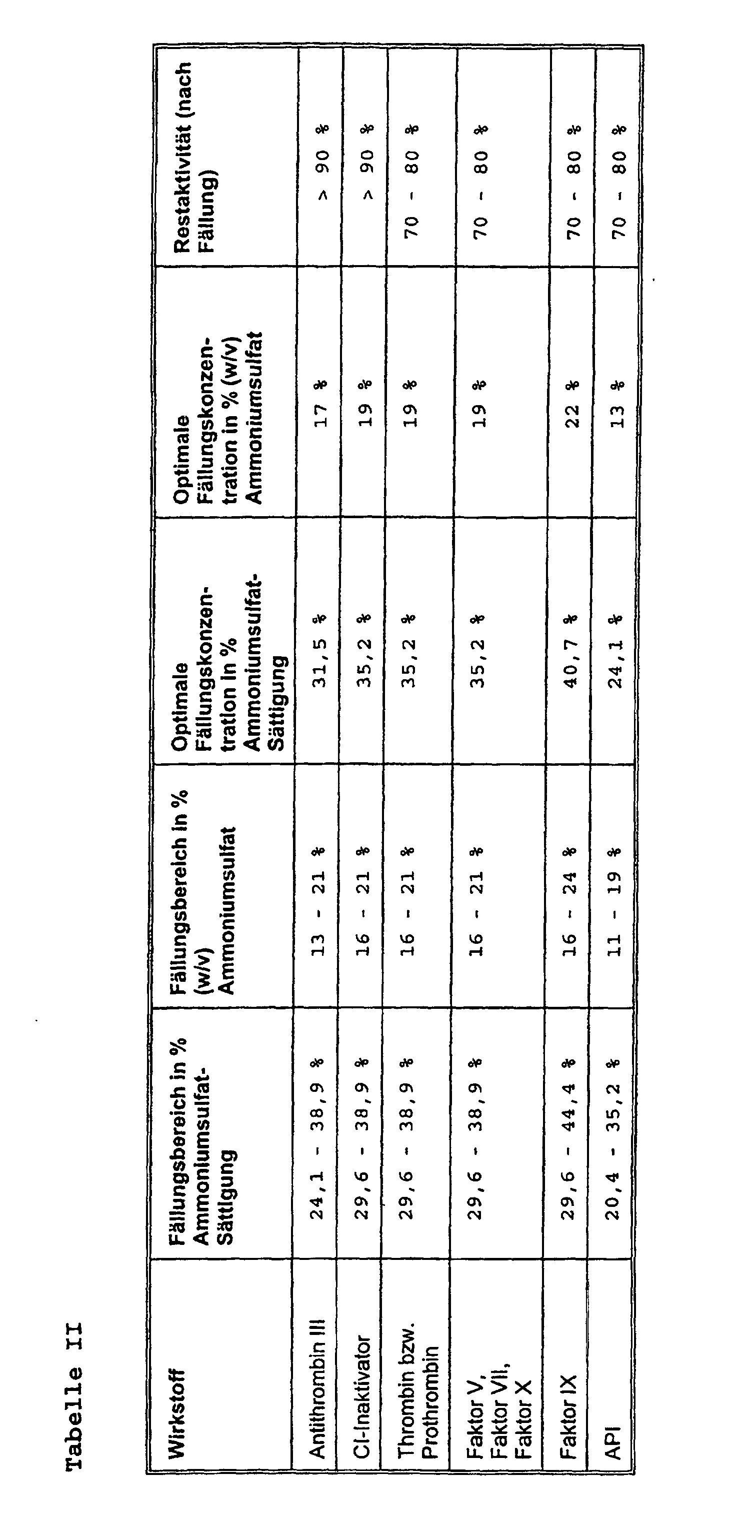

Gemäß Tabelle II gelten die Fällungsbedingungen von Antithrombin III auch für die Wirkstoffe C1-Inaktivator, Thrombin bzw. Prothrombin, Faktor V, Faktor VII, Faktor X. Für Faktor IX liegt die optimale Fällungskonzentration bei 22% (w/v) Ammoniumsulfat bzw. bei 40,7% Ammoniumsulfat-Sättigung.

Für α₁-Antitrypsin liegt die optimale Fällungskonzentration eher bei 13% (w/v) Ammoniumsulfat bzw. 24,1 % Ammoniumsulfat-Sättigung.

Zur Herstellung einer therapeutisch einsetzbaren Plasmaproteinzubereitung wird das bei dem erfindungsgemäßen Verfahren gewonnene Proteinpräzipitat als Trockensubstanz in Form einer mit dem Proteinpräzipitat angereicherten Plasmafraktion in eine Durchstechflasche eingebracht. Als Hilfsstoffe werden Aminoessigsäure, Natriumchlorid und Natriumcitrat eingesetzt. Der Durchstechflasche wird eine Ampulle mit 10 ml Wasser für Injektionszwecke, pyrogenfrei, beigegeben.

## Patentansprüche

1. Verfahren zur Inaktivierung und/oder Eliminierung von umhüllten und/oder nicht-umhüllten Viren aus einer Plasmaproteinlösung durch Zusatz eines Ammoniumsalzes bei alkalischen pH-Werten, **dadurch gekennzeichnet, dass** man eine Plasmaproteinlösung bei Raumtemperatur und bei einer Ammoniumsalzkonzentration von 13 bis 22 Gew.-% einer Inkubation unterwirft, nach Abtrennung der Ausfällungen und Entfernung des Ammoniumsalzes bis auf einen Restgehalt von weniger als 0,5 mol/l mehrere Stunden bei etwa 60°C pasteurisiert und sie dann zu einer therapeutisch einsetzbaren Plasmaproteinzubereitung verarbeitet.

2. Verfahren nach Anspruch 1, **dadurch gekennzeichnet, dass** man nach der Inkubation mit einer Ammoniumsalzkonzentration von 13 bis 22 Gew.-% die Ammoniumsalzkonzentration auf 24 bis 27 Gew.-% erhöht, erneut inkubiert, das dabei entstehende virusfreie Präzipitat nach Entfernung des Ammoniumsalzes bis auf einen Restgehalt von weniger als 0,5 mol/l mehrere Stunden bei etwa 60°C pasteurisiert und es dann zu einer therapeutisch einsetzbaren Plasmaproteinzubereitung verarbeitet.

3. Verfahren nach den Ansprüchen 1 und 2, **dadurch gekennzeichnet, dass** man als Plasmaproteinlösung eine durch Affinitätschromatographie vorgereinigte Plasmaproteinlösung einsetzt.

4. Verfahren nach den Ansprüchen 1 bis 3, **dadurch gekennzeichnet, dass** es bei pH-Werten zwischen 8 und 11 durchgeführt wird.

5. Verfahren nach den Ansprüchen 1 bis 4, **dadurch gekennzeichnet, dass** die erste und die zweite Inkubation jeweils zwei bis vier Stunden durchgeführt werden.

6. Verfahren nach den Ansprüchen 1 bis 5, **dadurch gekennzeichnet, dass** als Ammoniumsalz Ammoniumsulfat eingesetzt wird.

7. Verfahren nach den Ansprüchen 1 bis 6, **dadurch gekennzeichnet, dass** die Pasteurisierung in Gegenwart von Saccharose und Glycin oder in Gegenwart von Saccharose und Kaliumacetat als Stabilisatoren durchgeführt wird.

8. Verfahren nach den Ansprüchen 1 bis 7, **dadurch gekennzeichnet, dass** es mit natürlichen oder biotechnologisch hergestellten Plasmaproteinen durchgeführt wird.

## Claims

1. A process for the inactivation and/or elimination of coated and/or noncoated viruses from a plasma protein solution by addition of an ammonium salt at alkaline pHs, **which comprises** subjecting a plasma protein solution to incubation at room temperature and at an ammonium salt concentration of 13 to 22% by weight, pasteurizing it at approximately 60°C for several hours after separation of the precipitates and removal of the ammonium salt down to a residual content of less than 0.5 mol/l and then processing it to give a therapeutically employable plasma protein preparation.

2. The process as claimed in claim 1, **wherein,** after incubation with an ammonium salt concentration of 13 to 22% by weight, the ammonium salt concentration is increased to 24 to 27% by weight, the mixture is incubated again, the virus-free precipitate resulting in this way is pasteurized at approximately 60°C for several hours after removal of the ammonium salt down to a residual content of less than 0.5 mol/l and then processed to give a therapeutically employable plasma protein preparation.

3. The process as claimed in claims 1 and 2, **wherein** the plasma protein solution employed is a plasma protein solution which is prepurified by affinity chromatography.

4. The process as claimed in claims 1 to 3, **wherein** the process is carried out at pHs of between 8 and 11.

5. The process as claimed in claims 1 to 4, **wherein** the first and the second incubation are in each case carried out for two to four hours.

6. The process as claimed in claims 1 to 5, **wherein** the ammonium salt employed is ammonium sulfate.

7. The process as claimed in claims 1 to 6, **wherein** the pasteurization is carried out in the presence of sucrose and glycine or in the presence of sucrose and potassium acetate as stabilizers.

8. The process as claimed in claims 1 to 7, **wherein** the process is carried out using natural or bio-technologically produced plasma proteins.

## Revendications

1. Procédé d'inactivation et/ou d'élimination de virus avec enveloppe et/ou sans enveloppe d'une solution de protéine plasmatique par addition d'un sel d'ammonium à pH alcalin, **caractérisé en ce que** l'on soumet à incubation, une solution de protéine plasmatique à la température ambiante et à une concentration en sel d'ammonium de 13 à 22% en poids, on stérilise après séparation des précipitations et élimination du sel d'ammonium, jusqu'à une teneur résiduelle inférieure à 0,5 mole/litre, pendant plusieurs heures à environ 60°C et on traite alors en une préparation de protéine plasmatique pouvant être mise en oeuvre thérapeutiquement.

2. Procédé selon la revendication 1, **caractérisé en ce que** l'on augmente, après l'incubation avec une concentration en sel d'ammonium de 13 à 22% en poids, la concentration en sel d'ammonium jusqu'à 24 à 27% en poids, on incube à nouveau, le précipité exempt de virus formé est stérilisé après élimination du sel d'ammonium, jusqu'à une teneur résiduelle inférieure à 0,5 mole/litre, pendant plusieurs heures à environ 60°C et on traite en une préparation de protéine plasmatique pouvant être mise en oeuvre thérapeutiquement.

3. Procédé selon les revendications 1 et 2, **caractérisé en ce que** l'on met en oeuvre comme solution de protéine plasmatique, une solution de protéine plasmatique prépurifiée par chromatographie d'affinité.

4. Procédé selon les revendications 1 à 3, **caractérisé en ce qu'**il est réalisé à un pH allant de 8 à 11.

5. Procédé selon les revendications 1 à 4, **caractérisé en ce que** les première et deuxième incubations sont réalisées chaque fois, pendant deux à quatre heures.

6. Procédé selon les revendications 1 à 5, **caractérisé en ce que** l'on met en oeuvre le sulfate d'ammonium comme sel d'ammonium.

7. Procédé selon les revendications 1 à 6, **caractérisé en ce que** l'on réalise la stérilisation en présence de saccharose et de glycine ou en présence de saccharose et d'acétate de potassium comme stabilisants.

8. Procédé selon les revendications 1 à 7, **caractérisé en ce qu'**il est réalisé avec des protéines plasmatiques naturelles ou produites par biotechnologie.
